Europäisches Patentamt

European Patent Office

Office européen des brevets

(19)

(11) Numéro de publication: **0 298 179 B1**

(12) **FASCICULE DE BREVET EUROPEEN**

(45) Date de publication de fascicule du brevet: **15.04.92**

(51) Int. Cl.5: **A61K 35/72**, A61K 35/78, A23L 1/304, //(A61K35/78,35:72, 35:60,33:00,31:20)

(21) Numéro de dépôt: **87401615.7**

(22) Date de dépôt: **09.07.87**

(54) Nouvelles compositions riches en oligoéléments et leurs procédés de fabrication.

(43) Date de publication de la demande:
**11.01.89 Bulletin 89/02**

(45) Mention de la délivrance du brevet:
**15.04.92 Bulletin 92/16**

(84) Etats contractants désignés:
**BE CH DE ES FR GB IT LI NL SE**

(56) Documents cités:
**EP-A- 0 113 608
FR-A- 2 513 517
FR-A- 2 600 890**

**DICTIONNAIRE VIDAL, 1981, page 1167, O.V.P., Paris, FR;**

**ROTE LISTE, 1965, page 772, Editio Cantor, Aulendorf/Württ., DE;**

**Encyclopédia Larousse, Vol. 7 "oligoéléments"**

(73) Titulaire: **MOREAU, Pierre**
**10, rue Oswaldo Cruz**
**F-75016 Paris(FR)**

Titulaire: **Moatti, Roger Elie**
**13 rue Fortuny**
**F-75017 Paris(FR)**

Titulaire: **Schiepan, Marc**
**2 rue des Erables**
**F-78150 Rocquencourt(FR)**

(72) Inventeur: **MOREAU, Pierre**
**10, rue Oswaldo Cruz**
**F-75016 Paris(FR)**
Inventeur: **Moatti, Roger Elie**
**13 rue Fortuny**
**F-75017 Paris(FR)**
Inventeur: **Schiepan, Marc**
**2 rue des Erables**
**F-78150 Rocquencourt(FR)**

(74) Mandataire: **Orès, Bernard et al**
**Cabinet ORES 6, Avenue de Messine**
**F-75008 Paris(FR)**

## Description

La présente invention est relative à de nouvelles compositions riches en oligoéléments et à leurs procédés de fabrication.

La nécessité pour l'homme des oligoéléments est une notion actuellement universellement reconnue ; leur absence ou leur insuffisance se traduit par un défaut de croissance ou de développement qui peut s'accompagner de symptômes spécifiques de carences. Certains parmi les oligoéléments et notamment le fer, le cobalt, le nickel, le cuivre, le chrome, le manganèse, le molybdène, le fluor, le vanadium, l'étain, le zinc, l'iode et le sélénium constituent des oligo-éléments essentiels indispensables à la vie. Ils sont impliqués dans de très nombreuses fonctions de l'organisme telles : la croissance (pour le Zn, le F, le Mn, le Cu, le Se, le V), la reproduction (Zn, Mn), la dentition (F, Mo, V), la gustation (Cu, Zn, Ni), l'érythropoïèse (Fe, Zn,Cu, Co, Mo), la coagulation (Mn, Cu, Zn), le métabolisme glucidique (Cr, Mn,Zn), le métabolisme lipidique (Cr, V, Zn, Cu), le métabolisme protéique (Zn), le fonctionnement cérébral (Mn, Cu, Zn, Fe), la lutte contre les radicaux libres (Zn, Se, Mn, Cu,),etc...

Aussi de nombreuses préparations (dietétiques ou galéniques) contenant les oligoéléments et notamment les oligoéléments essentiels, ont été préconisées et mises sur le marché sous forme de poudres, de comprimés, de solutions.

Toutefois, si la biodisponibilité qui représente la fraction de l'apport total d'un oligoélément disponible pour une fonction biologique, dépend des divers phénomènes existant au niveau du transport plasmatique, de la captation et du stockage cellulaire, elle dépend surtout du rendement d'absorption à partir des aliments (cf. par exemple, Dictionnaire Vidal, 1981, "Spasmag" ou EP-A-0 113 608).

Des travaux récents ont démontré que l'assimilation des oligoéléments ne se fait correctement que lorsque l'apport est effectué sous forme organique et principalement sous forme de sels avec un acide aminé porteur de thiol comme, par exemple, la méthionine.

La culture de saccharomyces contenant les oligoéléments conduit à la levure riche en groupes thiols capteurs d'oligoéléments et porteurs vers la cellule réceptrice. Plusieurs sortes de levures enrichies en oligoéléments sont actuellement disponibles sur le marché.

Les Inventeurs, en étudiant les différentes compositions disponibles par l'examen systématique des taux sanguins ont pu mettre au point une "nouvelle génération d'oligoéléments" pratiquement entièrement assimilable.

La présente invention a pour objet une nouvelle composition riche en oligoéléments, caractérisée en ce qu'elle est constituée de :

0,001 à 10 % d'oligoéléments pris dans le groupe constitué par le magnésium,le fer,le cobalt,le nickel, le cuivre,le chrome,le manganèse,le molybdène,le fluor,l'iode, le vanadium,le zinc,l'étain et le sélénium la proportion étant indiquée en poids rapporté au métal cité,

23 à 98 % d'un support protéique
2 à 75 % d'acides gras polyinsaturés et notamment des acides ω3 et ω6 polyinsaturés.

Selon un mode de réalisation particulièrement avantageux de l'objet de l'invention, la composition contient entre 10 et 20 % d'acides gras ω3 polyinsaturés.

Selon un mode de réalisation avantageux de l'objet de l'invention, les acides gras polyinsaturés sont ajoutés sous forme d'autolysat de poissons et/ou de laitance de poissons et/ou d'huile de poissons et/ou d'huiles végétales.

Selon un autre mode de réalisation avantageux de l'objet de l'invention, le support protéique est constitué par de la levure (saccharomyces cerevisiae ou levure de bière) et/ou par de la poudre de lait et/ou par de la poudre de soja et/ou par de l'extrait de viande.

La présente invention est également relative à un procédé de préparation de la composition conforme à la présente invention, caractérisé en ce que :

a) l'on autolyse les organes de poissons de mers froides pendant une période comprise entre 1 et 8 jours, à une température comprise entre 30 et 80°C et à un pH compris entre 3,5 et 7,8, jusqu'à obtention d'un organolysat dont le rapport

$$\frac{N \text{ aminé}}{N \text{ total}}$$

soit compris entre 15 et 45 %,

b) en ce que fermente à une température comprise entre 8 et 29°C pendant une période de 3 à 7 jours une levure (Saccharomyces cerevisiae ou levure de bière) enrichie avec 5 à 35 % d'oligoéléments désires, puis dans le groupe constitué par les sels pharmaceutiquement compatibles, des éléments indiqués plus haut, et en ce que

c) on concentre les produits de ces deux fermentations, on mélange dans des proportions désirées et on répartit selon le cas sous forme de gélules, capsules, comprimés, ampoules, Suspensions et sirops.

Il est évident que si on le désire, on peut obtenir les compositions conformes à la présente invention par mélange d'une source d'acides gras polyinsaturés (telle par exemple une huile végétale) avec un support protéique (la poudre de lait, par exemple), et une quantité appropriée d'oligoéléments désirés.

Outre les dispositions qui précèdent, l'invention comprend encore d'autres dispositions qui ressortiront de la description qui va suivre.

La présente invention vise particulièrement les compositions à usage diététique, pharmaceutique et vétérinaire, riches en oligoéléments essentiels et pratiquement totalement assimilables.

L'invention pourra être mieux comprise à l'aide du complément de description qui va suivre, qui se réfère à des exemples de préparation et à un compte-rendu de l'examen de la pénétration d'oligoéléments dans le corps humain.

Il doit être bien entendu, toutefois, que ces exemples et compte-rendu sont donnés uniquement à titre d'illustration de l'objet de l'invention, mais n'en constituent en aucune manière une limitation.

EXEMPLE 1

Dans un fermenteur réglé à 27°C, on cultive pendant 4 jours la levure de Saccharomyces cerevisiae enrichie avec 25% de son poids en oligoéléments constitués par le Zn, le Se, le Mo et le Mn. A la fin de la fermentation, on concentre sous vide à une température inférieure à 75°C.

EXEMPLE 2

Dans un fermenteur réglé à 45°C et à un pH de 6,1, on autolyse les viscères et organes de poissons (salmonidés, thons, sardines et harengs) pendant 4 Jours. A la fin de cette période, on vérifie si la rapport

$$\frac{\text{N aminé}}{\text{N total}}$$

est compris entre 15 et 45 %. Si tel est le cas, on arrête la fermentation, on filtre et on concentre sous vide.

EXEMPLE 3

On mélange finement les poudres obtenues selon les exemples 1 et 2, on sèche l'ensemble à l'air chaud à 30°C et on répartit en forme galénique désirée.

EXEMPLE 4

On mélange l'huile de poissons pêchés en mer froide à température inférieure à 16°C contenant environ 45% d'un mélange d'acide docosahexaénoïque et d'acide eicosapentaénoïque, c'est-à-dire de la série $\omega$3 avec de l'huile végétale (telle que l'huile de germes de blé ou l'huile de colza, par exemple), riche en acide gras polyinsaturés de la série $\omega$6.

On ajoute à ce mélange de la levure de bière enrichie en oligoéléments puis de l'eau distillée en quantité suffisante pour réaliser après homogénéisation, une émulsion stable qui peut avantageusement se répartir en capsules, suppositoires, pommades, crèmes, etc...

EXEMPLE 5

A un mélange d'huiles de poissons tel que décrit dans l'exemple 4, on ajoute de la poudre de lait et des oligo éléments sous forme organique comme, par exemple, la sélénométhionine et le chrome fixé sur "Glucose Tolérance Factor" jusqu'à obtention d'une pâte homogène. Cette pâte est ensuite répartie en capsules de gélatine.

COMPTE-RENDU D'EXAMEN DE LA PENETRATION D'OLIGOELEMENTS DANS LE SANG

A. Cas de sélénium

Dans le Tableau I sont résumés les résultats comparatifs obtenus après 5 semaines du traitement avec :

- le sélénite de sodium
- la levure de Saccharomyces cerevisiae enrichie en selenium
- le produit conforme à la présente invention.

Le taux moyen initial du sujet carencé est 0,17 ± 0,02 ppm (les chiffres indiqués représentent une moyenne des résultats obtenus sur 10 sujets).

## TABLEAU I

| Durée de l'administration : 5 semaines  Dose journalière : 150 $\mu$ g de SE | Taux sanguins moyens |
|---|---|
| Sélénite de Na | 0,18 ± 0,02 |
| Levure de Saccharomyces cerevisiae enrichie en Selenium | 0,23 ± 0,02 |
| Produit conforme à la présente invention | 0,32 ± 0,02 |

B. Cas du Zinc

Les mesures effectuées portaient sur le taux de zinc dans les cheveux après deux mois de traitement avec une dose de 20 mg de zinc (exprimé en Zn métal) par jour, sous forme de :

- sulfate de zinc
- gluconate de zinc
- la levure de Saccharomyces enrichie en zinc
- produit conforme à la présente invention

Le Tableau II résume les résultats obtenus (le taux moyen initial du sujet carencé est de 100 ppm ± 0,5).

## TABLEAU II

| Durée du traitement : 2 mois<br>Dose journalière : 20 mg de Zn | Taux de Zn dans les cheveux (en ppm) |
| --- | --- |
| Sulfate de zinc | 120 ppm ± 0,5 |
| Gluconate de zinc | 140 ppm ± 0,5 |
| Levure enrichie en zinc | 155 ppm ± 0,5 |
| Produit conforme à la présente invention | 180 ppm ± 0,5 |

**Revendications**

**Revendications pour les Etats contractants suivants : BE, CH, DE, FR, GB, IT, LI, NL, SE**

1.  Nouvelle composition riche en oligoéléments, caractérisée en ce qu'elle est constituée de :
    0,001 à 10 % en poids d'oligoéléments pris dans le groupe constitué par le magnésium, le fer, le cobalt, le nickel, le cuivre, le chrome, le manganèse, le molybdène, le fluor, l'iode, le vanadium, le zinc, l'étain et le sélénium,
    la proportion étant indiquée en poids rapporté au métal cité,
    23 à 98 % d'un support protéique,
    2 à 75 % d'acides gras polyinsaturés et notamment des acides $\omega 3$ et $\omega 6$ polyinsaturés.

2.  Composition selon la Revendication 1, caractérisée en ce qu'elle contient entre 10 et 20 % d'acides gras $\omega 3$ polyinsaturés.

3.  Composition selon la Revendication 1, caractérisée en ce que les acides gras polyinsaturés sont ajoutés sous forme d'autolysat de poissons et/ou de laitance de poissons et/ou d'huile de poissons et/ou d'huiles végétales.

4.  Composition selon la Revendication 1, caractérisée en ce que le support protéique est constitué par de la levure (Saccharomyces cerivisiae ou levure de bière) et/ou par de la poudre de lait et/ou par de la poudre de soja et/ou par de l'extrait de viande.

5.  Procédé de préparation de la composition selon l'une quelconque des Revendications 1 à 4, caractérisé en ce que :
    a) l'on autolyse les organes de poissons de mers froides pendant une période comprise entre 1 et 8 jours, à une température comprise entre 30 et 80°C et à un pH compris entre 3,5 et 7,8 jusqu'à à obtention d'un organolysat dont le rapport

$$\frac{\text{N aminé}}{\text{N total}}$$

soit compris entre 15 et 45 %,

b) en ce que l'on fermente à une température comprise entre 8 et 29 pendant une période de 3 à 7 jours une levure (Saccharomyces cerevisiae ou levure de bière) enrichie avec 5 à 35 % en poids d'oligoéléments désirés,pris dans le groupe constitué par les sels pharmaceutiquement compatibles des éléments : le magnésium, le fer, le cobalt, le nickel, le cuivre, le chrome, le manganèse, le molybdène, le fluor, l'iode, le vanadium, le zinc, l'étain et le sélénium, le pourcentage indiqué étant en poids rapporté au métal, et en ce que

c) on concentre les produits de ces deux fermentations, on mélange dans des proportions désirées et on répartit selon le cas, sous forme de gélules, capsules, comprimés, ampoules, suspensions et sirops.

6. Produits diététiques, pharmaceutiques ou vétérinaires, caractérisés en ce qu'ils contiennent les compositions selon l'une quelconque des revendications 1 à 4.

**Revendications pour l'Etat contractant suivant : ES**

1. Procédé de préparation d'une composition riche en oligoéléments, constituée de :
   - 0,001 à 10 % en poids d'oligoéléments pris dans le groupe constitué par le magnésium, le fer, le cobalt, le nickel, le cuivre, le chrome, le manganèse, le molybdène, le fluor, l'iode, le vanadium, le zinc, l'étain et le sélénium, la proportion étant indiquée en poids rapporté au métal cité,
   - 23 à 98 % d'un support protéique,
   - 2 à 75 % d'acides gras polyinsaturés et notamment des acides $\omega 3$ et $\omega 6$ polyinsaturés,
   
   caractérisé en ce qu'il comprend le mélange de 2 à 75 % d'une source d'acides gras polyinsaturés avec 23 à 98 % d'un support protéique et 0,001 à 10 % d'oligoéléments désires.

2. Procédé selon la revendication 1, caractérisé en ce qu'on incorpore de 10 à 20 % d'acides gras $\omega 3$ insaturés.

3. Procédé selon la revendication 1, caractérisé en ce que les acides gras polyinsaturés sont ajoutés sous forme d'autolysat de poissons et/ou de laitance de poissons et/ou d'huile de poissons et/ou d'huiles végétales.

4. Procédé selon la revendication 1, caractérisé en ce que le support protéique est constitué par de la levure (*Saccharomyces cerevisiae* ou levure de bière) et/ou par de la poudre de lait et/ou par de la poudre de soja et/ou par de l'extrait de viande.

5. Procédé de préparation d'une composition riche en oligoéléments, constituée de :
   - 0,001 à 10 % en poids d'oligoéléments pris dans le groupe constitué par le magnésium, le fer, le cobalt, le nickel, le cuivre, le chrome, le manganèse, le molybdène, le fluor, l'iode, le vanadium, le zinc, l'étain et le sélénium,
   la proportion étant indiquée en poids rapporté au métal cité,
   - 23 à 98 % d'un support protéique,
   - 2 à 75 % d'acides gras polyinsaturés et notamment des acides $\omega 3$ et $\omega 6$ polyinsaturés,
   
   caractérisé en ce que :
   a) l'on autolyse les organes de poissons de mers froides pendant une période comprise entre 1 et 8 jours, à une température comprise entre 30 et 80°C et à un pH compris entre 3,5 et 7,8, jusqu'à obtention d'un organolysat dont le rapport

$$\frac{\text{N aminé}}{\text{N total}}$$

soit compris entre 15 et 45 %,
b) en ce que l'on fermente à une température comprise entre 8 et 29°C pendant une période de 3 à 7 jours une levure (Saccharomyces cerevisiae ou levure de bière) enrichie avec 5 à 35 % en poids d'oligoéléments désirés,pris dans le groupe constitué par les sels pharmaceutiquement compatibles des éléments : le magnésium, le fer, le cobalt, le nickel, le cuivre, le chrome, le manganèse, le molybdène, le fluor, l'iode, le vanadium, le zinc, l'étain et le sélénium, le pourcentage indiqué étant en poids rapporté au métal, et en ce que

c) on concentre les produits de ces deux fermentations, on mélange dans des proportions désirées et on répartit selon le cas, sous forme de gélules, capsules, comprimés, ampoules, suspensions et sirops.

**Claims**
**Claims for the following Contracting States : BE, CH, DE, FR, GB, IT, LI, NL SE**

1. New composition rich in trace elements, characterized in that it consists of:
   - 0.001 to 10% by weight of trace elements taken from the group made up of magnesium, iron, cobalt, nickel, copper, chromium, manganese, molybdenum, fluorine, iodine, vanadium, zinc, tin and selenium, the proportion being given by weight with respect to the metal indicated,
   - 23 to 98% of a protein support,
   - 2 to 75% of polyunsaturated fatty acids and especially of $\omega 3$ and $\omega 6$ polyunsaturated fatty acids.

2. Composition as claimed in Claim 1, characterized in that it contains between 10 and 20% of $\omega 3$ polyunsaturated fatty acids.

3. Composition as claimed in Claim 1, characterized in that the polyunsaturated fatty acids are added in the form of fish autolysate and/or milt and/or fish oil and/or vegetable oils.

4. Composition as claimed in Claim 1, characterized in that the protein support consists of yeast (*Saccharomyces cerevisiae* or brewer's yeast) and/or milk powder and/or soya powder and/or meat extract.

5. Process for preparation of the composition as claimed in any one of Claims 1 to 4, characterized in that:
   (a) the organs of cold-sea fish are autolysed for a period of 1 to 8 days, at a temperature between 30 and 80 °C and a pH between 3.5 and 7.8, until an organolysate is obtained in which the ratio $N_{amino}/N_{total}$ is between 15 and 45%,
   (b) a yeast (*Saccharomyces cerevisiae* or brewer's yeast) is fermented at a temperature between 8 and 29 °C for a period of 3 to 7 days, enriched with 5 to 35% of the desired trace elements taken from the group made up of the pharmaceutically-compatible salts of the following elements: magnesium, iron, cobalt, nickel, copper, chromium, manganese, molybdenum, fluorine, iodine, vanadium, zinc, tin and selenium, the percentage being indicated by weight with respect to the metal, and in that
   (c) the products of these two fermentations are concentrated, mixed in desired proportions, and distributed as required in the form of capsules, tablets, ampules, suspensions and syrups.

6. Dietetic, pharmaceutical or veterinary products, characterized in that they contain the compositions as claimed in anyone of Claims 1 to 4.

**Claims for the following Contracting State : ES**

1. Process for the preparation of a composition rich in trace elements, consisting of:
   - 0.001 to 10% by weight of trace elements taken from the group made up of magnesium, iron, cobalt, nickel, copper, chromium, manganese, molybdenum, fluorine, iodine, vanadium, zinc, tin and selenium, the proportion being given by weight with respect to the metal indicated,
   - 23 to 98% of a protein support,
   - 2 to 75% of polyunsaturated fatty acids and especially of $\omega 3$ and $\omega 6$ polyunsaturated fatty acids,
   characterized in that it comprises the mixture of 2 to 75% of a source of polyunsaturated fatty acids with 23 to 98% of a protein support and 0.001 to 10% of the desired trace elements.

2. Process as claimed in Claim 1, characterized in that between 10 and 20% of $\omega 3$ polyunsaturated fatty acids are incorporated in said composition.

3. Process as claimed in Claim 1, characterized in that the polyunsaturated fatty acids are added in the form of fish autolysate and/or milt and/or fish oil and/or vegetable oils.

**4.** Process as claimed in Claim 1, characterized in that the protein support consists of yeast (*Saccharomyces cerevisiae* or brewer's yeast) and/or milk powder and/or soya powder and/or meat extract.

**5.** Process for the preparation of a composition rich in trace elements, consisting of:
- 0.001 to 10% by weight of trace elements taken from the group made up of magnesium, iron, cobalt, nickel, copper, chromium, manganese, molybdenum, fluorine, iodine, vanadium, zinc, tin and selenium, the proportion being given by weight with respect to the metal indicated,
- 23 to 98% of a protein support,
- 2 to 75% of polyunsaturated fatty acids and especially of $\omega 3$ and $\omega 6$ polyunsaturated fatty acids,

characterized in that:

(a) the organs of cold-sea fish are autolysed for a period of 1 to 8 days, at a temperature between 30 and 80 °C and a pH between 3.5 and 7.8, until an organolysate is obtained in which the ratio $N_{amino}/N_{total}$ is between 15 and 45%,

(b) a yeast (*Saccharomyces cerevisiae* or brewer's yeast) is fermented at a temperature between 8 and 29 °C for a period of 3 to 7 days, enriched with 5 to 35% of the desired trace elements taken from the group made up of the pharmaceutically-compatible salts of the following elements: magnesium, iron, cobalt, nickel, copper, chromium, manganese, molybdenum, fluorine, iodine, vanadium, zinc, tin and selenium, the percentage being indicated by weight with respect to the metal, and in that

(c) the products of these two fermentations are concentrated, mixed in desired proportions, and distributed as required in the form of capsules, tablets, ampules, suspensions and syrups.

**Patentansprüche**

**Patentansprüche für folgende Vertragsstaaten : BE, CH, DE, FR, GB, IT, LI, NL, SE**

**1.** Neue Zusammensetzung, die reich an Oligoelementen ist, dadurch gekennzeichnet, daß sie besteht aus

0,001 bis 10 Gew.-% Oligoelementen, ausgewählt aus der Gruppe Magnesium, Eisen, Kobalt, Nickel, Kupfer, Chrom, Mangan, Molybdän, Fluor, Jod, Vanadin, Zink, Zinn und Selen, wobei der Mengenanteil in Gew.-% auf das genannte Metall bezogen ist,

23 bis 98 Gew.-% eines Protein-Trägers und

2 bis 75 Gew.-% mehrfach ungesättigte Fettsäuren und insbesondere mehrfach ungesättigten 3$\omega$- und 6$\omega$-Säuren.

**2.** Zusammensetzung nach Anspruch 1, dadurch gekennzeichnet, daß sie zwischen 10 und 20 Gew.-% mehrfach ungesättigte 3$\omega$-Fettsäuren enthält.

**3.** Zusammensetzung nach Anspruch 1, dadurch gekennzeichnet, daß die mehrfach ungesättigten Fettsäuren in Form eines Fischautolysats und/oder von Fischmilch und/oder von Fischöl und/oder von pflanzlichen Ölen zugegeben worden sind.

**4.** Zusammensetzung nach Anspruch 1, dadurch gekennzeichnet, daß der Protein-Träger besteht aus Hefe (Saccharomyces cerevisiae oder Bierhefe) und/oder Milchpulver und/oder Sojapulver und/oder Fleischextrakt.

**5.** Verfahren zur Herstellung der Zusammensetzung nach einem der Ansprüche 1 bis 4, dadurch gekennzeichnet, daß man

(a) die Organe von Fischen von kalten Meeren für einen Zeitraum zwischen 1 und 8 Tagen bei einer Temperatur zwischen 30 und 80°C und bei einem pH-Wert zwischen 3,5 und 7,8 autolysiert bis zum Erhalt eines Organolysats, dessen Verhältnis Amin-N : Gesamt-N zwischen 15 und 45 % liegt,

(b) eine Hefe (Saccharomyces cerevisiae oder Bierhefe), die angereichert ist mit 5 bis 35 Gew.-% der gewünschten Oligoelemente, ausgewählt aus der Gruppe der pharmazeutisch verträglichen Salze der Elemente Magnesium, Eisen, Kobalt, Nickel, Kupfer, Chrom, Mangan, Molybdän, Fluor, Jod, Vanadin, Zink, Zinn und Selen, wobei der genannten Gewichtsprzentsatz auf das Metall bezogen ist, bei einer Temperatur zwischen 8 und 29°C für eine Zeitspanne von 3 bis 7 Tagen fermentiert und

(c) die Produkte dieser beiden Fermentationen konzentriert, in den gewünschten Mengenverhältnissen mischt und je nach den Umständen in Form von Gelen, Kapseln, Tabletten, Ampullen, Suspensionen und Sirupen dosiert.

6. Diätetische, pharmazeutische oder Veterinär-Produkte, dadurch gekennzeichnet, daß sie die Zusammensetzungen nach einem der Ansprüche 1 bis 4 enthalten.

**Patentansprüche für folgenden Vertragsstaat : ES**

1. Verfahren zur Herstellung einer Zusammensetzung, die reich an Oligoelementen ist und besteht aus
   - 0,001 bis 10 Gew.-% Oligoelementen, ausgewählt aus der Gruppe Magnesium, Eisen, Kobalt, Nickel, Kupfer, Chrom, Mangan, Molybdän, Fluor, Jod, Vanadin, Zink, Zinn und Seien, wobei der Mengenanteil in Gew.-% auf das genannte Metall bezogen ist,
   - 23 bis 98 Gew.-% eines Protein-Trägers und
   - 2 bis 75 Gew.-% mehrfach ungesättigten Fettsäuren und insbesondere mehrfach ungesättigten 3ω-und 6ω-Säuren,
   dadurch gekennzeichnet, daß es umfaßt das Mischen von 2 bis 75 Gew.-% einer Quelle für mehrfach ungesättigte Säuren mit 23 bis 98 Gew.-% eines Protein-Trägers und 0,001 bis 10 Gew.-% der gewünschten Oligoelemente.

2. Verfahren nach Anspruch 1, dadurch gekennzeichnet, daß man 10 bis 20 Gew.-% ungesättigte 3ω-Fettsäuren einarbeitet.

3. Verfahren nach Anspruch 1, dadurch gekennzeichnet, daß die mehrfach ungesättigten Fettsäuren in Form eines Fischautclysats und/oder von Fischmilch und/oder von Fischöl und/oder von pflanzlichen Ölen zugegeben werden.

4. Verfahren nach Anspruch 1, dadurch gekennzeichnet, daß der Protein-Träger aus Hefe (Saccharomyces cerevisiae oder Bierhefe) und/oder Milchpulver und/oder Sojapulver und/oder Fleischextrakt besteht.

5. Verfahren zur Herstellung einer Zusammensetzung, die reich an Oligoelementen ist und besteht aus
   - 0,001 bis 10 Gew.-% Oligoelementen, ausgewählt aus der Gruppe Magnesium, Eisen, Kobalt, Nickel, Kupfer, Chrom, Mangan, Molybdän, Fluor, Jod, Vanadin, Zink, Zinn und Selen, wobei der Mengenanteil in Gew.-% auf das genannte Metall bezogen ist,
   - 23 bis 98 Gew.-% eines Protein-Trägers und
   - 2 bis 75 Gew.-% mehrfach ungesättigten Fettsäuren und insbesondere mehrfach ungesättigten 3 - und 6 -Säuren,
   dadurch gekennzeichnet, daß man
   (a) die Organe von Fischen von kalten Meeren während einer Zeitspanne zwischen 1 und 8 Tagen bei einer Temperatur zwischen 30 und 80°C und bei einem pH-Wert zwischen 3,5 und 7,8 autolysiert bis zum Erhalt eines Organolysats, dessen Verhältnis Amin-N : Gesamt-N zwischen 15 und 45 % liegt,
   (b) eine Hefe (Saccharomyces cerevisiae oder Bierhefe), die angereichert ist mit 5 bis 35 Gew.-% der gewünschten Oligoelemente, ausgewählt aus der Gruppe der pharmazeutisch verträglichen Salze der Elemente Magnesium, Eisen, Kobalt, Nickel, Kupfer, Chrom, Mangan, Molybdän, Fluor, Jod, Vanadin, Zink, Zinn und Selen, wobei der genannte Gewichtsprozentsatz auf das Metall bezogen ist, bei einer Temperatur zwischen 8 und 29°C während einer Zeitspanne von 3 bis 7 Tagen fermentiert und
   (c) die Produkte dieser beiden Fermentationen konzentriert, in den gewünschten Mengenverhältnissen mischt und sie je nach den Umständen in Form von Gelen, Kapseln, Tabletten, Ampullen, Suspensionen und Sirupen dosiert.